Europäisches Patentamt

European Patent Office

Office européen des brevets

(19)

(11) Numéro de publication : **0 531 204 A1**

(12) # DEMANDE DE BREVET EUROPEEN

(21) Numéro de dépôt : **92402387.2**

(22) Date de dépôt : **02.09.92**

(51) Int. Cl.$^5$ : **C12N 9/02,** C12N 15/53,
C07K 3/02, A61K 37/02,
A61K 7/00, A61K 49/00

(30) Priorité : **05.09.91 FR 9110973**

(43) Date de publication de la demande :
**10.03.93 Bulletin 93/10**

(84) Etats contractants désignés :
**AT BE CH DE GB IT LI SE**

(71) Demandeur : **COMMISSARIAT A L'ENERGIE
ATOMIQUE
31-33, rue de la Fédération
F-75015 Paris (FR)**

(72) Inventeur : **Fromageot, Pierre
6 avenue de la Reine Amélie
F-78150 Le Chesnay (FR)**
Inventeur : **Genet, Roger
9 allée des Arpents
F-91470 Limours (FR)**

(74) Mandataire : **Orès, Bernard et al
Cabinet ORES 6, Avenue de Messine
F-75008 Paris (FR)**

(54) Préparation enzymatique purifiée issue de chromobacterium violaceum, son procédé d'obtention et son application à la production d'alpha, bêta déhydrotryptophanyl-peptides.

(57) Préparation enzymatique purifiée issue de *Chromobacterium violaceum*, présentant une activité déshydrogénasique, son procédé d'obtention et son application à la production d'$\alpha,\beta$-déhydrotryptophanyl-peptides.

Ledit procédé de préparation d'$\alpha,\beta$-déhydrotryptophanyl-peptides et d'$\alpha,\beta$-déhydrotryptophanyl-protéines, est caractérisé en ce que l'on met en contact :

— un peptide ou une protéine à transformer, et

— une préparation enzymatique purifiée ayant une activité de L-tryptophane déshydrogénase et présentant une activité spécifique comprise entre 35 et 45 unités.secondes$^{-1}$ par mole d'hème pour le N-acétyle-L-tryptophanamide (NATA), un poids moléculaire apparent de l'ordre de 680 kDa, un point isoélectrique de l'ordre de 4, une température optimale d'activité supérieure à 80°C et une stabilité à la température,

à un pH compris entre 3 et 8 et à une température comprise entre 20° et 60°C.

Applications des déhydropeptides et des déhydroprotéines obtenus commune sondes, réactifs, médicaments ou produits cosmétologiques.

EP 0 531 204 A1

La présente invention est relative à une préparation enzymatique purifiée issue de *Chromobacterium violaceum*, présentant une activité déshydrogénasique, à son procédé d'obtention et à son application à la production d'α,β-déhydrotryptophanyl-peptides.

Une activité déshydrogénasique, agissant sur la chaîne latérale du tryptophane a été décrite dans Methods in Enzymology (1987, 142, 195-217 ; K. TAKAI et O. HAYAISHI). La *tryptophan side chain oxidase* (TSO), isolée de *Pseudomonas* XA (ATCC 29574), est un système multienzymatique qui catalyse la déshydrogénation en $C_\alpha$ et $C_\beta$ de résidus tryptophanyles. Cependant, le composé insaturé (-ΔTrp-) n'est qu'un produit secondaire dans le catabolisme du tryptophane, formé en équilibre avec les produits de dégradation (β-hydroxy et β-céto), à partir d'un intermédiaire unique indolyloxazoline et ne permet pas de maîtriser convenablement la production des composés déhydro du fait de la variation du rendement de synthèse de ces composés, notamment en fonction de la nature du substrat et des conditions expérimentales (le pH, en particulier). De plus, cette enzyme est faiblement spécifique et transforme le L-tryptophane (L-Trp), le D-tryptophane (D-Trp), les résidus dont le noyau indole est modifié ou substitué (5 OH-Trp) et les résidus décarboxylés (tryptamine).

P.J. DAVIS et al. ont mis en évidence l'existence d'une biotransformation du N-benzyloxycarbonyle-L-tryptophane dans une culture de bactérie à Gram négatif, *Chromobacterium violaceum* (ATCC 12472), se traduisant également par la formation d'une double liaison entre les carbones $C_\alpha$ et $C_\beta$ du L-tryptophane (BBA, 1975, 385, 133-144) ; ils ont également étudié la transformation de l'acide indole-3 propionique en acide indole-3 acrylique (J. Bacteriol., 1976, 126, 1, 544-546) et la stéréochimie de la déshydrogénation de la chaîne latérale du N-benzyloxycarbonyle-(S)-tryptophane (J.C.S. Chem. Comm., 1977, 821, 842-843).

Toutefois, cette réaction n'a été mise en évidence que sur des substrats particuliers dont la seule caractéristique commune est la présence d'un noyau indole, et ce uniquement lors de l'incubation de cellules au repos et dans des cultures de bactéries.

Il résulte de ce qui précède qu'aussi bien les réactions décrites dans TAKAI et al. que celles décrites dans DAVIS et al. ne permettent pas leur application à la production de déhydropeptides et de déhydroprotéines purs.

La présente invention s'est en conséquence donné pour but de pourvoir à une préparation enzymatique purifiée, présentant une activité déshydrogénasique spécifique du résidu L-tryptophane, à partir de *Chromobacterium violaceum* qui répond mieux aux besoins de la pratique que les préparations de l'Art antérieur, notamment en ce qu'elle permet d'obtenir des déhydropeptides ou des déhydroprotéines purs, dans des conditions opératoires faciles à mettre en oeuvre industriellement.

La présente invention a pour objet, un procédé de préparation d'α,β-déhydrotryptophanyl-peptides et d'α,β-déhydrotryptophanyl-protéines, caractérisé en ce que l'on met en contact :

- un peptide ou une protéine à transformer, et
- une préparation enzymatique purifiée ayant une activité de L-tryptophane déshydrogénase et présentant une activité spécifique comprise entre 35 et 45 unités.secondes$^{-1}$ par mole d'hème pour le N-acétyle-L-tryptophanamide (NATA), un poids moléculaire apparent de l'ordre de 680 kDa, un point isoélectrique de l'ordre de 4, une température optimale d'activité supérieure à 80°C et une stabilité à la température,

à un pH compris entre 3 et 8 et à une température comprise entre 20°et 60°C.

Selon un mode de mise en oeuvre avantageux dudit procédé, la transformation est effectuée en présence de catalase (EC 1.11.1.6), à une concentration finale de 50 à 100 μg/ml.

Selon un autre mode de mise en oeuvre avantageux dudit procédé, ladite préparation enzymatique purifiée est issue de *Chromobacterium violaceum*.

Les Inventeurs ont appelé cette préparation enzymatique purifiée L-tryptophane 2′,3′-oxydase (L-tryptophane:oxygène 2′,3′-oxydoréductase, EC 1.3.3.?), compte-tenu de ses propriétés fonctionnelles.

De manière avantageuse et surprenante, le déhydrotryptophane est le produit unique de la réaction enzymatique catalysée par la L-Trp-2′,3′-oxydase de *Chromobacterium violaceum*, quelles que soient les conditions de la réaction entre pH 3 et pH 8 ; de plus, le composé déhydro obtenu est stable lorsque le groupe α-aminé est impliqué dans une liaison peptidique ou protégé de manière convenable.

Egalement de manière inattendue, dans les conditions du procédé conforme à l'invention, on obtient des déhydropeptides et des déhydroprotéines dont tous les résidus tryptophanyles accessibles ont été transformés et ce avec un rendement de l'ordre de 100 % ; de plus, les composés déhydro n'entraînent pas nécessairement un changement drastique de leur activité biologique.

La création d'une double liaison en position $C_\alpha$-$C_\beta$ des résidus tryptophanyles introduit une contrainte conformationnelle qui confère à la molécule les autres propriétés suivantes :

- inhibition de la protéolyse par les principales endo- et exoprotéases : α-chymotrypsine, carboxy-peptidases A et Y, aminopeptidase M ;
- modification du spectre de fluorescence, qui permet notamment d'étudier spécifiquement les interactions portant sur un résidu tryptophanyle particulier de la molécule.

Les déhydropeptides et les déhydroprotéines obtenus par le procédé conforme à l'invention sont particulièrement stables et trouvent notamment application :
- dans la préparation de traceurs, grâce à l'introduction d'atomes stables ou radioactifs ($^2$D, $^3$H, $^{125}$I...), en un site spécifique de la molécule, tels que des sondes de photoaffinité, des sondes de fluorescence ou des sondes pour la résonance magnétique nucléaire (RMN),
- comme réactifs pour l'étude des interactions entre protéines ou l'étude topologique d'un récepteur ou d'un site actif,
- comme réactifs de diagnostic, y compris en imagerie médicale,
- comme nouvelles molécules (agonistes, antagonistes, inhibiteurs d'enzymes de conversion ou tout autre médicament), particulièrement intéressantes notamment du fait de leur stabilité plus grande (inhibition de la dégradation protéolytique, permettant une action prolongée et une meilleure réabsorption du peptide modifié), ou
- comme produits cosmétologiques.

La présente invention a également pour objet une préparation enzymatique purifiée ayant une activité de L-tryptophane-déshydrogénase, susceptible d'être mise en oeuvre dans un procédé de préparation d'$\alpha,\beta$-déhydrotryptophanyl-peptides et -protéines conforme à l'invention, caractérisée :
- en ce qu'elle présente :
. une activité spécifique comprise entre 35 et 45 unités.secondes$^{-1}$ par mole d'hème pour le N-acétyle-L-tryptophanamide (NATA),
. un poids moléculaire apparent de l'ordre de 680 kDa,
. un point isoélectrique de l'ordre de 4,
. une température optimale d'activité supérieure à 80°C ;
- en ce qu'elle est thermostable ; et
- en ce qu'elle catalyse la conversion de résidus tryptophanyles en résidus déhydrotryptophanyles à un pH compris entre 3 et 8.

Elle présente une structure protomérique de 88 kDa comprenant deux sous-unités, respectivement de 14 kDa et de 74 kDa.

Selon un mode de réalisation avantageux de ladite préparation, elle est susceptible d'être obtenue par extraction convenable à partir de *Chromobacterium violaceum*, laquelle extraction comprend une étape d'incubation dans un milieu dénaturant, de la fraction enzymatique isolée.

Selon un autre mode de réalisation avantageux de ladite préparation, elle est susceptible d'être obtenue par expression de l'information génétique, obtenue par clonage du gène ou de l'ADNc, dans un microorganisme approprié.

La préparation enzymatique purifiée conforme à l'invention présente des caractéristiques qui permettent son utilisation industrielle :
1. la formation d'$\alpha,\beta$-déhydrotryptophanyl-peptides et d'$\alpha,\beta$-déhydrotryptophanyl-protéines résulte directement de la catalyse ;
2. ces déhydropeptides et ces déhydroprotéines sont les produits uniques de la réaction enzymatique, quelles que soient les conditions opératoires entre pH 3 et pH 8, à condition que le groupement $\alpha$-aminé du résidu $\Delta$Trp soit impliqué dans une liaison peptidique ou protégé de manière convenable ;
3. l'enzyme possède une haute spécificité pour les résidus tryptophanyles : (i) au niveau du cycle indolique, qui ne doit être ni substitué ni modifié, (ii) au niveau de la fonction $\alpha$-carbonyle, dont la présence est indispensable à la catalyse, et enfin (iii) une stéréospécificité au niveau du carbone asymétrique ($C_\alpha$) : la réaction nécessite l'énantiomère L du tryptophane.

La présente invention a également pour objet un procédé d'obtention de ladite préparation enzymatique purifiée, caractérisé en ce qu'il comprend :
I - une étape de préparation de la fraction enzymatique brute Q1 qui comprend :
a. l'extraction des protéines totales par broyage des cellules de *Chromobacterium violaceum*, en présence d'un inhibiteur de l'activité protéolytique intrinsèque, ultracentrifugation et séparation des protéines par précipitation fractionnée dans un tampon approprié, et
b. l'obtention de la fraction enzymatique Q1, par une succession d'étapes chromatographiques appropriées, et
II - une étape de préparation de la fraction enzymatique purifiée Z1 par
c. une incubation de la fraction enzymatique Q1 en milieu dénaturant, et
d. perméation de gel de la fraction active Q1 incubée.

Parmi les principaux inhibiteurs de l'activité protéolytique endogène, on peut citer le PMSF (fluorure de phényl méthane sulfonyle), l'EDTA (acide éthylène diamine tétracétique), la benzamidine, la pepstatine A, la leupeptine etc...

Selon un mode de mise en oeuvre avantageux dudit procédé, l'inhibiteur de l'activité protéolytique intrinsèque est de préférence un mélange de PMSF et d'EDTA.

De manière connue, la précipitation fractionnée est mise en oeuvre avec du sulfate d'ammonium ou d'autres agents précipitants.

Selon encore un autre mode de mise en oeuvre avantageux dudit procédé, le milieu dénaturant comprend un agent dénaturant choisi dans le groupe qui comprend l'urée (3-4 M) et le SDS (dodécylsulfate de sodium) (0,1 à 2 % p/v), associé à un agent réducteur choisi dans le groupe qui comprend le dithiothréitol (DTT, >50 mM) et le 2-mercapto-éthanol (1 à 5 % v/v).

On définit, au sens de la présente invention, le milieu dénaturant commune un milieu qui détruit les interactions de forte ou faible énergie au sein des protéines, à l'exception de l'enzyme L-Trp-2',3'-oxidase ; ceci a pour effet une diminution du poids moléculaire apparent des protéines contaminantes, et une meilleure séparation en chromatographie de perméation de gel, l'activité enzymatique étant stable dans ces conditions.

Outre les dispositions qui précèdent, l'invention comprend encore d'autres dispositions, qui ressortiront de la description qui va suivre, qui se réfère à des exemples de mise en oeuvre du procédé objet de la présente invention.

Il doit être bien entendu, toutefois, que ces exemples sont donnés uniquement à titre d'illustration de l'objet de l'invention, dont ils ne constituent en aucune manière une limitation.

**EXEMPLE 1: Préparation de la L-Trp-2',3'-oxydase de _Chromobacterium violaceum_ (préparation enzymatique purifiée).**

1) Purification :

- La L-tryptophane déshydrogénase (L-Trp DH) ou L-Trp-2',3'-oxydase est préparée à partir de cellules de _Chromobacterium violaceum_ (ATCC 12472), cultivées en milieu de Mitoma, supplémenté en L-tryptophane à 0,5 g/l (DAVIS et al., BBA, 1975, 385, 133-144) en fermenteur de 50 litres, puis stockées après congélation à -80°C.

- La purification, dont le protocole est détaillé ci-après et sur le schéma 1, comprend une séparation des protéines par broyage, suivie d'une ultracentrifugation et d'une précipitation fractionnée au sulfate d'ammonium, l'obtention de la fraction enzymatique brute Q1 par une succession d'étapes chromatographiques et l'obtention de la fraction enzymatique purifiée Z1 par incubation de la fraction enzymatique Q1 en milieu dénaturant et perméation de gel de ladite fraction.

De manière plus précise :

- les bactéries _Chromobacterium violaceum_ stockées à -80°C sont décongelées dans un tampon A (Tris-HCl 0,1 M pH 7,0, EDTA 5 mM, PMSF 100 mg/l, CHAPS 1 %, DTT 10 mM) à une concentration de 1 ml/g et maintenues à une température de 4°C, puis on les centrifuge pendant 10 minutes à 5 000 g et à 4°C.

- le culot bactérien est repris dans du tampon A tel que défini ci-dessus, additionné de 5 % de glycérol (environ 0,4 ml/g) et le broyage des bactéries est réalisé sur ce culot en presse d'Eaton, à -60°C.

- lorsque le broyage est réalisé, on décongèle à nouveau, puis on centrifuge 20 minutes à 39 000 g (4°C).

- le surnageant de cette centrifugation (surnageant S1) est ultracentrifugé 1 heure à 100 000 g et à 4°C.

- le surnageant de cette ultracentrifugation (surnageant S2) est incubé pendant 1 heure à 30°C, en présence de Benzonase® (MERCK) 25 U/ml et de $MgCl_2$ 10 mM, afin de lyser le matériel nucléaire (ADN, ARN).

- une précipitation au sulfate d'ammonium à 20 % de saturation est réalisée sur le lysat obtenu, puis les fractions sont séparées par centrifugation à 39 000 g et à 4°C pendant 20 minutes.

- le surnageant de cette dernière centrifugation (surnageant S3) est précipité au sulfate d'ammonium à 45 % de saturation et centrifugé à 39 000 g et à °C pendant 20 minutes.

- on récupère le culot (C45 %), qui est solubilisé dans un tampon B (L-His HCl 20 mM pH 5,6), filtré sur cartouche Sep-pak ACCELL CM® (Waters) et rincé dans ce tampon B.

- la solution obtenue est soumise à une chromatographie d'adsorption sur colonne échangeuse d'anions Protein Pak® DEAE 8HR (Waters) et élution en tampon B (gradient 0, 2 à 1 M NaCl).

- l'éluat comprend la fraction enzymatique active Q1, qui est incubée avec de l'urée 3 M et du DTT 170 mM, pendant 30 min à 25°C.

- après incubation, la solution est soumise à une perméation de gel sur une colonne ZORBAX GF 450® (Du Pont de Nemours) suivie d'une élution en tampon C ($K_2HPO_4$ 0,2 M pH 7), débit 1 ml/min.

- la colonne est équilibrée grâce à un mélange standard de protéines dont le poids moléculaire est connu : immunoglobuline M (900 000), thyroglobuline (670 000), $\gamma$ globuline (158 000), ovalbumine (44 000), myoglobine (17 000), cyanocobalamine (1 350) ; l'activité enzymatique se trouve alors éluée sous forme d'un pic unique de protéine (Z1), dont le poids moléculaire apparent est estimé à 680 kDa et qui peut être stocké à -80°C (20 % glycérol).

## 2) Pureté de la préparation :

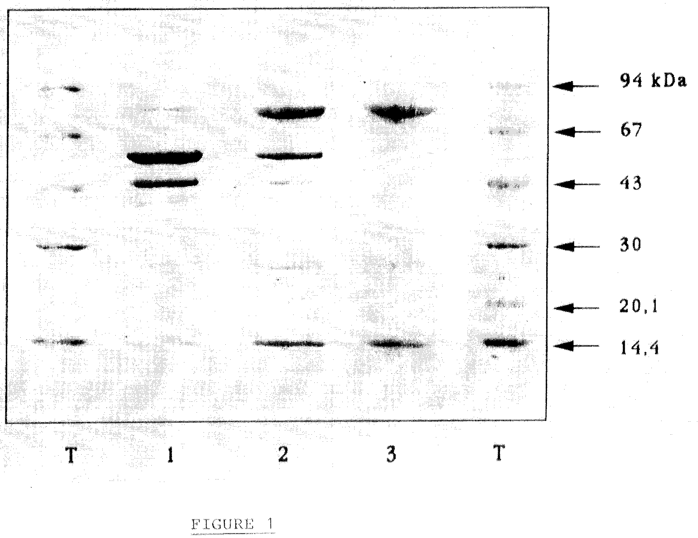

. Une électrophorèse en milieu dénaturant (SDS-PAGE) montre que la fraction Z1 est constituée de deux bandes majoritaires : 74 et 14 kDa (figure 1).

Cette figure 1 représente le suivi de la purification de la L-Trp 2′,3′-oxydase par électrophorèse en gel de polyacrylamide (gradient 8-18 %), contenant du dodécylsulfate de sodium (1 g/l), pH 6,4 et révélation au bleu de Coomassie ; les profils T sont des témoins de migration : phosphorylase b (94 kDa), sérumalbumine bovine (67 kDa), ovalbumine (43 kDa), anhydrase carbonique (30 kDa), inhibiteur de la trypsine (20,1 kDa) et $\alpha$-lactalbumine (14,4 kDa) ; le puits 1 correspond à la fraction C45 % ($\approx$ 50 $\mu$g) ; le puits 2 correspond à la fraction Q1 ($\approx$ 50 $\mu$g) et le puits 3 correspond à la fraction Z1 ($\approx$ 20 $\mu$g) pure.

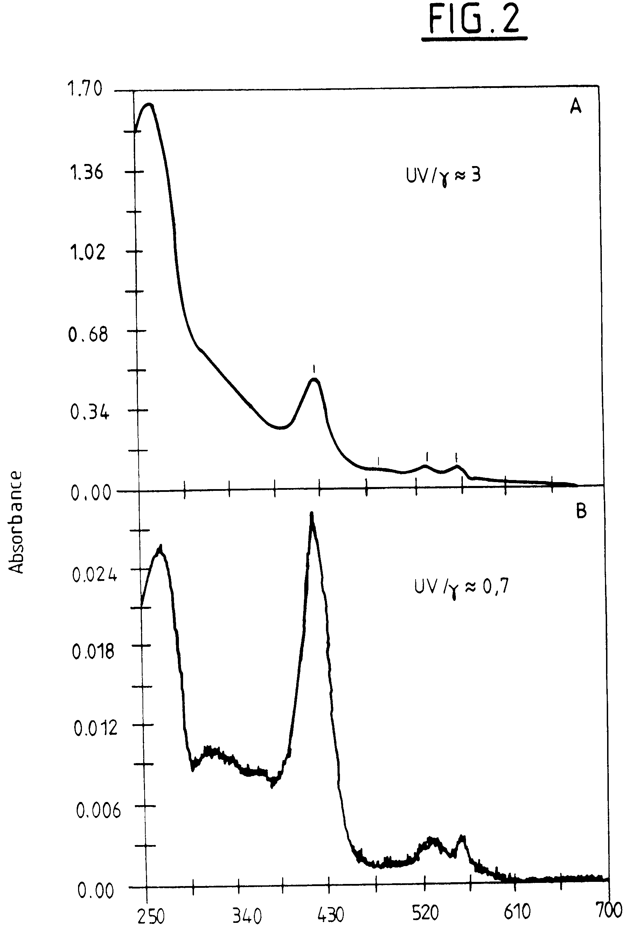

. Le spectre de la fraction enzymatique Z1 met en évidence la présence d'un cofacteur hémique (figure 2). La pureté de la préparation peut être estimée à partir du rapport UV/$\gamma$ des absorbances à 275 nm (région des aromatiques) et à 427 nm (raie Soret ($\gamma$) de l'hème oxydé). En effet, ce rapport diminue au cours de la purification et passe d'environ 3 après chromatographie d'échange d'ions (Q1) à 0,7 après perméation de gel (Z1, pureté >95 %). De cette valeur, on peut déduire par approximation le coefficient d'extinction molaire de l'hème oxydé à 427 nm, soit $\varepsilon_{427} \approx 120$ mM$^{-1}$.cm$^{-1}$, qui permet de calculer la concentration d'enzyme dans la préparation.

Le rendement global de purification s'élève à environ 6,5 mg de L-Trp-2′,3′-oxydase pour 100 grammes de cellules humides.

Les figures 2A et 2B illustrent les spectres de la L-Trp-2′,3′-oxydase ; la figure 2A correspond à la fraction Q1, l'enzyme ($\approx$ 2,7 $\mu$M) étant en solution dans un tampon L-histidine 20 mM, NaCl 0,2 M, pH 5,6 ; la figure 2B correspond à la fraction pure Z1, l'enzyme ($\approx$ 0,18 $\mu$M) étant en solution dans un tampon K$_2$HPO$_4$ 0,2 M, pH 7. Ces figures présentent en abscisse la longueur d'onde et en ordonnée l'absorbance.

## 3) Stockage :

Les conditions optimales de stabilité sont obtenues en tampon Bis-tris 0,1 M, pH 7. Les fractions Q1 et Z1 sont conservées congelées à -80°C.

## 4) Mesure de l'activité enzymatique :

Les mesures d'activité spécifique de l'enzyme ainsi que l'ensemble des expériences de cinétique à l'état stationnaire, ont été réalisées dans les conditions standard définies comme suit :

Température : 30°C
Substrat : N-acétyle-L-tryptophanamide (NATA) 1 mM
Tampon : succinate 50 mM, pH 5,5

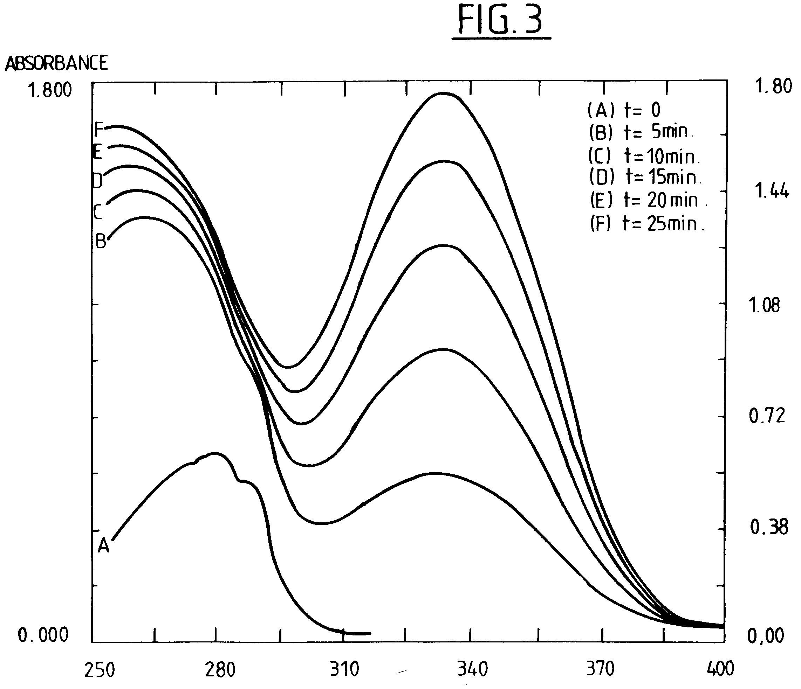

en suivant directement l'apparition du produit déhydro à 330 nm (figure 3). L'activité spécifique (As) de la fraction Q1 est calculée sur la base d'un coefficient d'extinction molaire à 330 nm de 18,8 mM$^{-1}$.cm$^{-1}$ déterminé au laboratoire (contre $\varepsilon_{330}$ = 19,89 mM$^{-1}$.cm$^{-1}$ dans la littérature (TAKAI et al. précité). Le NATA, qui représente le plus petit enchaînement peptidique possible, est utilisé comme substrat modèle de la réaction enzymatique. L'activité spécifique de la préparation (As) est estimée à 39,5 $\pm$ 3,1 unités pour le NATA (une unité enzymatique représentant une mole de produit formé par seconde par mole d'hème).

Cette figure 3, qui comporte en abscisse les longueurs d'onde et en ordonnée l'absorbance, illustre la cinétique de formation de la double liaison en C$_\alpha$-C$_\beta$ (déshydrogénation du N-acétyle-L-tryptophanamide), dans les conditions opératoires précisées ci-dessus (NATA 0,1 mM).

## 5) Caractérisation de l'enzyme:

- Structure : la L-tryptophane-2′,3′-oxydase est une hémoprotéine dont le poids moléculaire apparent avoisine 680 kDa, comme précisé ci-dessus. Elle est globalement acide : son point isoélectrique (pI) est d'environ 4. C'est un hétéropolymère, constitué de deux sous-unités en quantités stoechiométriques, 74 et 14 kilodaltons, assemblées sous forme d'un protomère de 88 kDa. Les protomères sont vraisemblablement les sous-unités fonctionnelles de l'enzyme, constituant à eux seuls une véritable chaîne de transfert d'électrons, et assemblés sous forme "holo-octamérique".

- Fonction : la L-tryptophane 2′,3′-oxydase catalyse la conversion du N-acétyle-L-tryptophanamide

(NATA) en $\alpha,\beta$-déhydroNATA aux dépens de l'oxygène, avec formation d'un équivalent de peroxyde d'hydrogène ($H_2O_2$).

La réaction enzymatique s'exprime à un pH optimum de $5 \pm 0,5$, tandis que la zone de stabilité optimale de l'enzyme est à pH $7 \pm 0,5$.

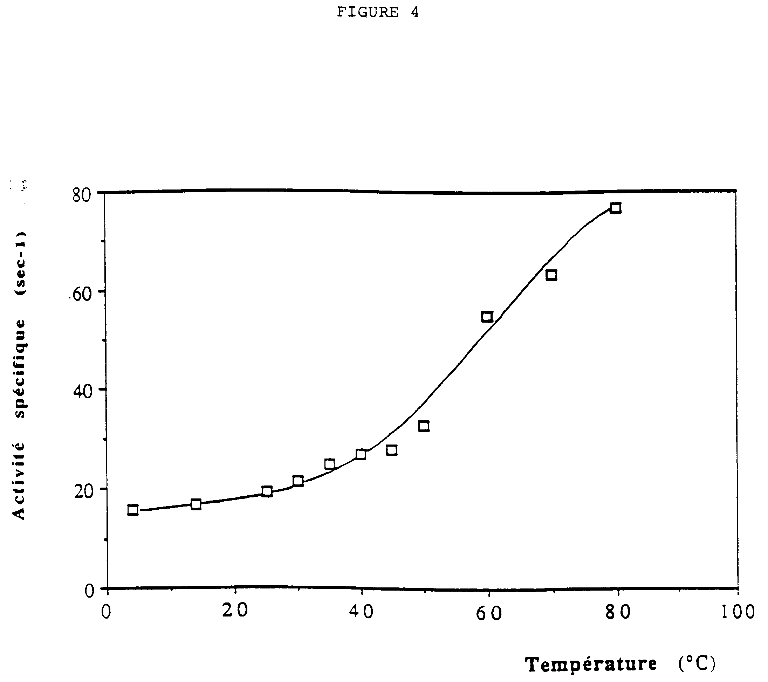

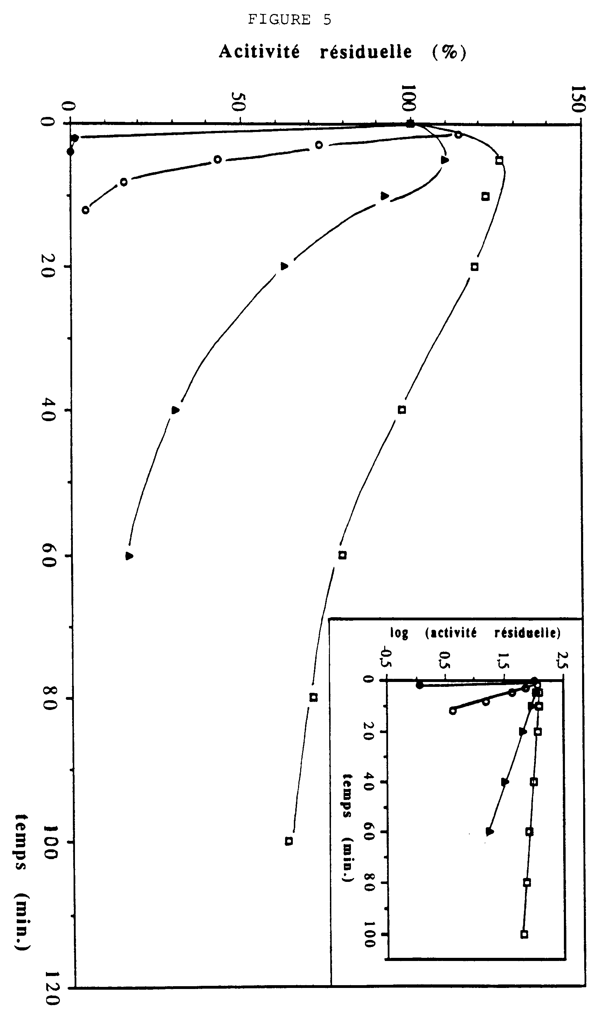

La température optimale de la cinétique se situe au dessus de 80°C (figure 4) ; cette figure 4, qui comporte en abscisse la température et en ordonnée l'activité spécifique, illustre l'effet de la température sur la cinétique ; l'enzyme est thermostable, caractérisée par une demi-vie évoluant de 90 minutes à 50°C à quelques secondes seulement au-delà de 80°C. La figure 5 montre que l'inactivation suit une cinétique du premier ordre, ce qui suggère que la dénaturation thermique de l'enzyme est bien le seul facteur responsable : l'enzyme "activée" (0,27 µM dans du bis-Tris 0,1 M, pH 7 conservée 3 h à température ambiante) est incubée à 50 (□), 60,5 (▲), 69 (○) et 80° C (●) ; l'activité enzymatique est mesurée en fonction du temps (test standard de cinétique).

6) Spécificité de la réaction:

Aucune transformation n'a pu être mise en évidence pour les aminoacides aromatiques : la N-acétyle-L-phénylalaninamide et la N-acétyle-L-tyrosinamide. Il en est de même lorsque le noyau indole est substitué : 5-hydroxyle L-Trp, N-formyle L-Trp et pour les isomères de la série D : D-tryptophane et N-acétyle D-tryptophane. Ces résultats montrent que l'enzyme possède une forte spécificité pour le noyau indole et pour l'énantiomère L (stéréospécificité).

De même, aucune réaction ne peut être mise en évidence sur la tryptamine, alors que l'acide indole-3 propionique est oxydé en acide indole-3 acrylique ; ces résultats démontrent que la fonction $\alpha$-aminée n'est pas impliquée dans la catalyse tandis que le groupement $\alpha$-carbonyle est absolument nécessaire.

7) Caractérisation et stabilité des produits de la réaction :

La déshydrogénation du NATA a été suivie en fonction du pH (pH 3 - 5,6 - 8), à une température de 30°C en tampon succinate 50 mM pH 5,6. Les produits de réaction sont analysés et purifiés en CLHP en phase inverse, puis lyophilisés et analysés en spectrométrie de masse (Désorption par Ionisation Chimique : DCI). Le $\Delta$NATA est le seul produit de la réaction enzymatique, caractérisé en CLHP (pic unique, $\lambda$max 333 nm) et en spectrophotométrie de masse ($MH^+=244$).

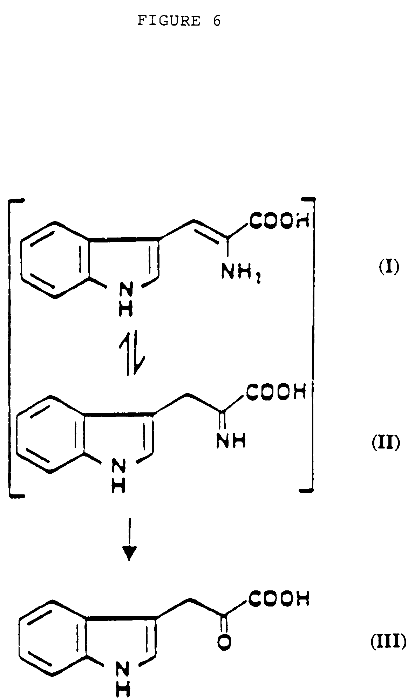

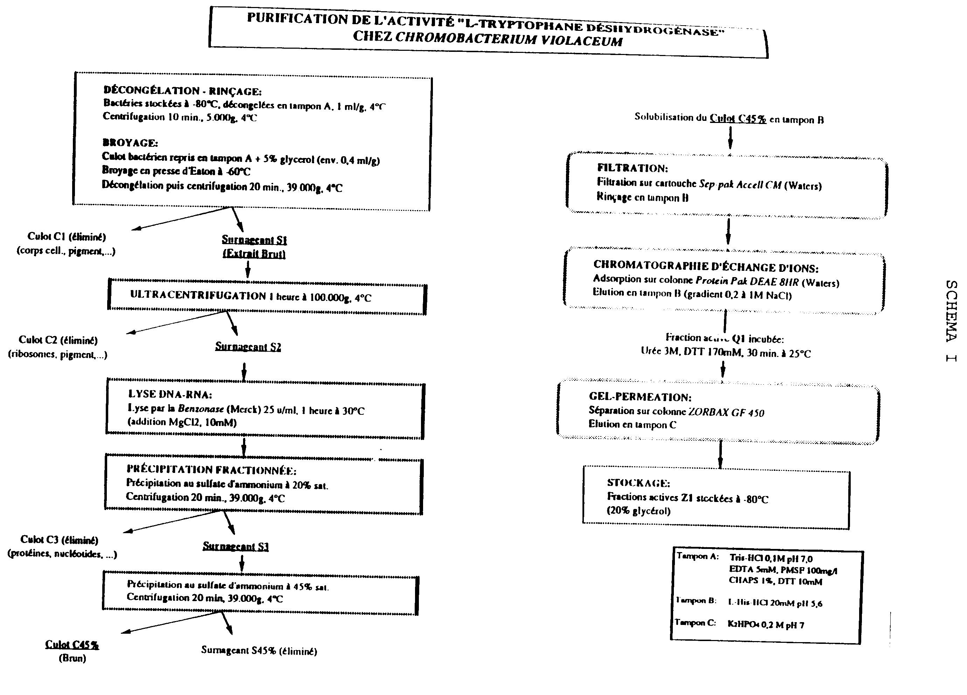

La conversion du L-tryptophane et du L-tryptophanamide, dont les fonctions $\alpha$-aminées respectives sont libres, se traduit par une augmentation de l'absorbance à 320 nm suivie après quelques minutes d'une phase de décroissance qui s'accompagne de l'inhibition totale de l'enzyme. L'analyse par spectrométrie de masse des produits de la réaction du $L$-$TrpNH_2$ met en évidence un pic moléculaire ($MH^+ = 203$) correspondant vraisemblablement au dérivé $\alpha$-céto : acide indole-3 pyruvique amide. Ce phénomène, est beaucoup plus rapide lorsque la fonction $\alpha$-carboxylique n'est pas substituée (L-Trp, comp. I), et se traduit par la formation d'une imine instable (II) qui s'hydrolyse pour donner naissance aux composés $\alpha$-céto (respectivement l'acide indole-3 pyruvique (III) et son homologue amide) -figure 6-. Il est donc préférable que la fonction $\alpha$-aminée soit bloquée, afin d'empêcher tout phénomène d'isomérisation et d'assurer une meilleure stabilité du composé déhydro.

**EXEMPLE 2: Procédé de préparation des déhydropeptides et déhydroprotéines.**

a) Peptides :

On procède comme à l'exemple 1.7).

Toutes les réactions ont été réalisées en présence de catalase. Dans chaque cas, les constantes cinétiques (Kcat et Km) sont calculées pour évaluer l'efficacité de l'enzyme pour chaque type de substrat. Les résultats sont présentés dans le Tableau I ci-après.

EP 0 531 204 A1

TABLEAU I

| SUBSTRATS | Nombre de résidus | SEQUENCE | Km ($\mu$M) | Kcat ($s^{-1}$) | Kcat/Km ($M^{-1}.s^{-1}$) |
|---|---|---|---|---|---|
| Penta-gastrine | 5 | Boc$\beta$AWMDF | 26 | 32 | $10^6$ |
| LHRH | 10 | pEHWSYGLRPG | 71 | 20 | $3.10^5$ |
| KKacoR 15 | 17 | KKKHWVYYTCCPDTPYL | 272 | 2 | $7.10^3$ |
| ACTH | 24 | SYSMEHFRWGKP VGKKRRPVKVYP | 930 | 17 | $2.10^4$ |
| $\beta$-lacto-globuline | 162 | $Trp^{19}-Trp^{51}$ | 2 % de transformation (*) | | |
| Catalase | 506 | $Trp^{14}-Trp^{142}-$ $Trp^{182}-Trp^{185}-$ $Trp^{276}-Trp^{302}$ | 12 % de transformation (*) | | |
| BSA | 582 | $Trp^{133}-Trp^{212}$ | 24 % de transformation (*) | | |

(*) : Valeurs données à titre indicatif, donnant une idée de la transformation du Trp dans les protéines en 24 heures, dans les conditions standard définies à l'exemple 1.7 pour le NATA (E/S $\approx$ 1/1000).

D'une façon générale l'efficacité de l'enzyme diminue avec la taille du peptide. Le Kcat reste cependant du même ordre de grandeur, sauf dans le cas du peptide KKacoR15 (peptide synthétique de la sous-unité $\alpha$ du récepteur nicotinique de l'acétylcholine) où elle est diminuée d'un facteur 10 ; ce résultat peut s'expliquer par la nature très hydrophobe, compacte, de ce peptide et la présence d'un pont disulfure. Le Km subit une variation plus importante pouvant atteindre un facteur 50 pour un peptide de 24 aminoacides. Dans tous les cas, quelle que soit la position du résidu tryptophanyle dans la séquence, les peptides testés jusqu'à 25 résidus ont été transformés avec succès ($\lambda$max 332 à 336 nm). Les rendements atteignent quasiment 100 %.

b) Protéines :

Comme précisé ci-dessus, l'accessibilité des résidus tryptophanyles, tant au niveau du noyau indolyle qu'au niveau des protons $C_\alpha$ et $C_\beta$, joue un rôle prépondérant. La transformation est très lente, cependant les rendements obtenus, dans les conditions standard, en 24 heures sur des protéines de grosse taille ($\beta$-lacto-globuline : 32 kDa, BSA : 67 kDa, catalase : 232 kDa) montrent que, dans des conditions adaptées (température, pH, milieu partiellement dénaturant...), la transformation des résidus tryptophanyles accessibles peut atteindre 100 %.

c) Propriétés particulières des déhydropeptides :

La création d'une double liaison en position $C_\alpha$-$C_\beta$ des résidus tryptophanyles introduit une contrainte conformationnelle qui confère à la molécule de nouvelles propriétés :
. Résistance à la protéolyse :
La présence de résidus $\alpha,\beta$-déhydrotryptophanyle au sein de chaînes polypeptidiques inhibe totalement la protéolyse par les principales endo- et exoprotéases : $\alpha$-chytmotrypsine, carboxypeptidases A et Y, aminopeptidase M. Les composés déhydro constituent donc un maillon de résistance dans la molécule, et pourraient permettre de prolonger l'action et de favoriser la réabsorption d'un peptide-médicament.

7

. Modification du spectre de fluorescence :

Les spectres de fluorescence du NATA et du ΔNATA ainsi que des principaux déhydro-peptides synthétisés ont été enregistrés. La présence de la double liaison provoque un déplacement de la longueur d'onde maximale d'émission qui passe de 372 nm (λexc. : 280 nm) à 427 nm (λexc. : 330 nm). Cette modification du spectre d'émission est donc particulièrement intéressante puisqu'elle permet d'étudier spécifiquement les interactions portant sur un résidu tryptophanyle particulier de la molécule.

. Activité biologique :

Les contraintes conformationnelles induites dans un peptide -ou une protéine- par la formation d'α,β-déhydrotryptophane, peuvent se traduire par des modifications structurales locales, susceptibles d'entraîner l'abolition ou au contraire l'exaltation de son activité biologique.

La [ΔTrp³] pentagastrine a été testée *in vivo* et les résultats montrent que ce peptide possède la même activité biologique que le peptide natif.

Il en ressort que la formation de déhydrotryptophane n'entraîne pas nécessairement un changement drastique de l'activité biologique des produits testés.

. Utilisation de la double liaison pour l'introduction de sondes spécifiques :

Les résidus déhydrotryptophanyles générés *in situ* au sein de peptides et de protéines constituent un site privilégié de marquage.

La réduction de la double liaison par le tritium gaz ($^3H_2$), en présence de catalyseur stéréospécifique a déjà été réalisée sur le modèle ΔNATA (PINTO-ALPHANDARY H. et al., J. Label. Comp. & Radiopharmaceuticals, 1988, 25, 1273-1279). Cette méthode présente de nombreux avantages du fait de sa spécificité, de la forte ressemblance de l'atome de tritium avec l'atome d'hydrogène -absence de modification de la structure primaire-, de la forte radioactivité spécifique de cet isotope (29 Ci/mat) et de sa demi-vie élevée (12,6 ans). L'addition d'iode froid ou radioactif (ICl, pH 7) sur la double liaison est une seconde possibilité. (MAELICKE et al., FEBS Lett., 1978, 85, 296-300).

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée de la présente invention.

**Revendications**

1°) Procédé de préparation d'α,β-déhydrotryptophanyl-peptides et d'α,β-déhydrotryptophanyl-protéines, caractérisé en ce que l'on met en contact :

- un peptide ou une protéine à transformer, et
- une préparation enzymatique purifiée ayant une activité de L-tryptophane déshydrogénase et présentant une activité spécifique comprise entre 35 et 45 unités.secondes⁻¹ par mole d'hème pour le N-acétyle-L-tryptophanamide (NATA), un poids moléculaire apparent de l'ordre de 680 kDa, un point isoélectrique de l'ordre de 4 et une température optimale d'activité supérieure à 80°C et une stabilité à la température, à un pH compris entre 3 et 8 et à une température comprise entre 20° et 60°C.

2°) Procédé selon la revendication 1, caractérisé en ce que la transformation est effectuée en présence de catalase (EC 1.11.1.6), à une concentration finale de 50 à 100 µg/ml.

3°) Procédé selon la revendication 1 ou la revendication 2, caractérisé en ce que ladite préparation enzymatique purifiée est issue de *Chromobacterium violaceum*.

4°) Préparation enzymatique purifiée ayant une activité de L-tryptophane déshydrogénase, susceptible d'être mise en oeuvre dans un procédé selon l'une quelconque des revendications 1 à 3, caractérisée :

- en ce qu'elle présente :
   . une activité spécifique comprise entre 35 et 45 unités.secondes⁻¹ par mole d'hème pour le N-acétyle-L-tryptophanamide (NATA),
   . un poids moléculaire apparent de l'ordre de 680 kDa,
   . un point isoélectrique de l'ordre de 4,
   . une température optimale d'activité supérieure à 80°C ;
- en ce qu'elle est thermostable ; et
- en ce qu'elle catalyse la conversion de résidus tryptophanyles en résidus déhydrotryptophanyles à un pH compris entre 3 et 8.

5°) Préparation enzymatique purifiée selon la revendication 4, caractérisée en ce qu'elle est susceptible d'être obtenue par extraction convenable à partir de *Chromobacterium violaceum*, laquelle extraction comprend une étape d'incubation dans un milieu dénaturant, de la fraction enzymatique isolée.

**6°)** Préparation enzymatique purifiée selon la revendication 4, caractérisée en ce qu'elle est susceptible d'être obtenue par expression de l'information génétique, obtenue par clonage du gène ou de l'ADNc, dans un microorganisme approprié.

**7°)** Procédé d'obtention de la préparation enzymatique purifiée selon l'une quelconque des revendications 4 à 6, caractérisé en ce qu'il comprend :

I - une étape de préparation de la fraction enzymatique brute Q1 qui comprend :

a. l'extraction des protéines totales par broyage des cellules de *Chromobacterium violaceum*, en présence d'un inhibiteur de l'activité protéolytique intrinsèque, ultracentrifugation et séparation des protéines par précipitation fractionnée dans un tampon approprié, et

b. l'obtention de la fraction enzymatique Q1, par une succession d'étapes chromatographiques appropriées, et

II - une étape de préparation de la fraction enzymatique purifiée Z1 par

c. une incubation de la fraction enzymatique Q1 en milieu dénaturant, et

d. perméation de gel de la fraction active Q1 incubée.

**8°)** Procédé selon la revendication 7, caractérisé en ce que l'inhibiteur de l'activité protéolytique intrinsèque est de préférence un mélange de PMSF et d'EDTA.

**9°)** Procédé selon la revendication 7 ou la revendication 8, caractérisé en ce que le milieu dénaturant comprend un agent dénaturant choisi dans le groupe qui comprend l'urée (3-4 M) et le SDS (dodécylsulfate de sodium) (0,1 à 2 % p/v), associé à un agent réducteur choisi dans le groupe qui comprend le dithiothréitol (DTT, >50 mM) et le 2-mercapto-éthanol (1 à 5 % v/v).

**10°)** Application des $\alpha,\beta$-déhydrotryptophanylpeptides et d'$\alpha,\beta$-déhydrotryptophanyl-protéines, obtenus par le procédé selon l'une quelconque des revendications 1 à 3, à la préparation de sondes froides et de traceurs radioactifs.

**11°)** Application des $\alpha,\beta$-déhydrotryptophanylpeptides et d'$\alpha,\beta$-déhydrotryptophanyl-protéines, obtenus par le procédé selon l'une quelconque des revendications 1 à 3, à la préparation de réactifs.

**12°)** Application des $\alpha,\beta$-déhydrotryptophanylpeptides et d'$\alpha,\beta$-déhydrotryptophanyl-protéines, obtenus par le procédé selon l'une quelconque des revendications 1 à 3, à la préparation de médicaments.

**13°)** Application des $\alpha,\beta$-déhydrotryptophanylpeptides et d'$\alpha,\beta$-déhydrotryptophanyl-protéines, obtenus par le procédé selon l'une quelconque des revendications 1 à 3, à la préparation de produits cosmétologiques.

FIGURE 1

FIG.2

# FIG.3

ABSORBANCE

(A) t = 0
(B) t = 5min.
(C) t = 10min.
(D) t = 15min.
(E) t = 20min.
(F) t = 25min.

EP 0 531 204 A1

FIGURE 4

FIGURE 5

Acitivité résiduelle (%)

FIGURE 6

(I)

(II)

(III)

# PURIFICATION DE L'ACTIVITÉ "L-TRYPTOPHANE DÉSHYDROGÉNASE" CHEZ *CHROMOBACTERIUM VIOLACEUM*

**DÉCONGÉLATION - RINÇAGE:**
Bactéries stockées à -80°C, décongelées en tampon A, 1 ml/g, 4°C
Centrifugation 10 min., 5.000g, 4°C

**BROYAGE:**
Culot bactérien repris en tampon A + 5% glycerol (env. 0,4 ml/g)
Broyage en presse d'Eaton à -60°C
Décongélation puis centrifugation 20 min., 39.000g, 4°C

Culot C1 (éliminé)
(corps cell., pigment,...)

Surnageant S1
(Extrait Brut)

**ULTRACENTRIFUGATION 1 heure à 100.000g, 4°C**

Culot C2 (éliminé)
(ribosomes, pigment,...)

Surnageant S2

**LYSE DNA-RNA:**
Lyse par la *Benzonase* (Merck) 25 u/ml, 1 heure à 30°C
(addition MgCl2, 10mM)

**PRÉCIPITATION FRACTIONNÉE:**
Précipitation au sulfate d'ammonium à 20% sat.
Centrifugation 20 min., 39.000g, 4°C

Culot C3 (éliminé)
(protéines, nucléotides, ...)

Surnageant S3

Précipitation au sulfate d'ammonium à 45% sat.
Centrifugation 20 min., 39.000g, 4°C

Culot C45%
(Brun)

Surnageant S45% (éliminé)

---

Solubilisation du Culot C45% en tampon B

**FILTRATION:**
Filtration sur cartouche *Sep-pak Accell CM* (Waters)
Rinçage en tampon B

**CHROMATOGRAPHIE D'ÉCHANGE D'IONS:**
Adsorption sur colonne *Protein Pak DEAE 8HR* (Waters)
Elution en tampon B (gradient 0,2 à 1M NaCl)

Fraction active Q1 incubée:
Urée 3M, DTT 170mM, 30 min. à 25°C

**GEL-PERMEATION:**
Séparation sur colonne *ZORBAX GF 450*
Elution en tampon C

**STOCKAGE:**
Fractions actives Z1 stockées à -80°C
(20% glycérol)

| Tampon A: | Tris-HCl 0,1M pH 7,0 |
| | EDTA 5mM, PMSF 100mg/l |
| | CHAPS 1%, DTT 10mM |
| Tampon B: | L-His-HCl 20mM pH 5,6 |
| Tampon C: | K2HPO4 0,2 M pH 7 |

16

## EP 0 531 204 A1

**Office européen des brevets**

## RAPPORT DE RECHERCHE EUROPEENNE

Numero de la demande

EP    92 40 2387
Page 1

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| D,A | FEBS LETTERS. vol. 85, no. 2, Janvier 1978, AMSTERDAM NL pages 296 - 300 A.MAELICKE ET AL 'An enzymatic approach to the labeling of tryptophan residues in peptides and proteins' * le document en entier * --- | 1,10-11 | C12N9/02 C12N15/53 C07K3/02 A61K37/02 A61K7/00 A61K49/00 |
| D,A | JOURNAL OF LABELLED COMPOUNDS AND RADIOPHARMACEUTICALS vol. 25, no. 11, 1988, BRUXELLES pages 1273 - 1279 H. PINTO-ALPHANDARY ET AL 'Asymetric titration of N-acetyl alpha-beta dehydrotrytophanamide' * l'article en entier ,plus particulierement les pages 1277 et 1278 * --- | 1,10 | |
| A | JOURNAL OF BIOLOGICAL CHEMISTRY. vol. 256, no. 15, Août 1981, BALTIMORE US pages 7834 - 7843 SEIJI ITO ET AL 'Enzymatic modification of tryptophan residues by tryptophan side chain oxidase I and  II from Pseudomonas' * le document en entier * --- | 1 | |
| A | JOURNAL OF BIOLOGICAL CHEMISTRY. vol. 259, no. 7, 10 Avril 1984, BALTIMORE US pages 4452 - 4457 TAKAI K. ET AL 'Enzymatic dehydrogenation of tryptophan residues of human globins by tryptophan side chain oxidase II' * le document en entier * --- -/-- | 1 | |

**DOMAINES TECHNIQUES RECHERCHES (Int. Cl.5 )**

C12N

Le présent rapport a été établi pour toutes les revendications

| Lieu de la recherche | Date d'achèvement de la recherche | Examinateur |
|---|---|---|
| LA HAYE | 01 DECEMBRE 1992 | LE CORNEC N.D.R. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
........................................................................
& : membre de la même famille, document correspondant

EPO FORM 1503 03.82 (P0402)

17

Office européen
des brevets

**RAPPORT DE RECHERCHE EUROPEENNE**

Numero de la demande

EP    92 40 2387
Page 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| D,A | BIOCHEMICA ET BIOPHYSICA ACTA vol. 385, 1975, AMSTERDAM;NL. pages 133 - 144 P.J. DAVIS ET AL 'Metabolism of N-carbobenzoxyl-L-tryptophan by Chromobacterium violaceum' * page 144 * | 1-4,6-8 | |

-----

DOMAINES TECHNIQUES RECHERCHES (Int. Cl.5 )

Le présent rapport a été établi pour toutes les revendications

| Lieu de la recherche | Date d'achèvement de la recherche | Examinateur |
|---|---|---|
| LA HAYE | 01 DECEMBRE 1992 | LE CORNEC N.D.R. |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons

................................................................................

& : membre de la même famille, document correspondant

EPO FORM 1503 03.82 (P0402)